# EUROPEAN PATENT SPECIFICATION

(11) **EP 1 677 821 B1**
(45) Date of publication and mention of the grant of the patent: **27.02.2013**
(21) Application number: 04795590.1
(22) Date of filing: 18.10.2004
(51) Int. Cl.: A61K 39/00, C12N 5/00

(54) **A METHOD FOR INCREASING CD8+ CYTOTOXIC T CELL REPONSES AND FOR TREATING MULTIPLE SCLEROSIS**
VERFAHREN ZUR VERSTÄRKUNG DER CD8+ ZYTOTOXISCHEN T-ZELL-ANTWORTEN UND ZUR BEHANDLUNG VON MULTIPLER SKLEROSE
PROCEDE POUR AUGMENTER DES REPONSES DE LYMPHOCYTES T CYTOTOXIQUES CD8+ ET POUR TRAITER UNE SCLEROSE EN PLAQUES

(30) Priority: 17.10.2003 US 512212 P
(43) Date of publication of application: 12.07.2006
(73) Proprietor: BAYLOR COLLEGE OF MEDICINE, Houston, TX 77030 (US)
(72) Inventor: ZANG, Ying, C., Q., Missouri City, TX 77459 (US)
(74) Representative: Walton, Seán Malcolm
(86) International application number: PCT/US2004/034448
(87) International publication number: WO 2005/037309

(56) References cited:
- WO-A-02/092773
- WO-A-03/024393
- WO-A1-2005/037309
- STEINMAN LAWRENCE: "Myelin-specific CD8 T cells in the pathogenesis of experimental allergic encephalitis and multiple sclerosis" JOURNAL OF EXPERIMENTAL MEDICINE, vol. 194, no. 5, 3 September 2001 (2001-09-03), pages F27-F30, XP002322201 ISSN: 0022-1007 cited in the application
- HUSEBY ERIC S ET AL: "A pathogenic role for myelin-specific CD8+ T cells in a model for multiple sclerosis" JOURNAL OF EXPERIMENTAL MEDICINE, vol. 194, no. 5, 3 September 2001 (2001-09-03), pages 669-676, XP002322200 ISSN: 0022-1007 cited in the application
- SUN D ET AL: "Myelin antigen-specific CD8+ T cells are encephalitogenic and produce severe disease in C57BL/6 mice." JOURNAL OF IMMUNOLOGY (BALTIMORE, MD. : 1950) 15 JUN 2001, vol. 166, no. 12, 15 June 2001 (2001-06-15), pages 7579-7587, XP002322202 ISSN: 0022-1767
- TSUCHIDA T ET AL: "AUTOREACTIVE CD8+ T-CELL RESPONSES TO HUMAN MYELIN PROTEIN-DERIVED PEPTIDES" PROCEEDINGS OF THE NATIONAL ACADEMY OF SCIENCES OF USA, NATIONAL ACADEMY OF SCIENCE. WASHINGTON, US, vol. 91, no. 23, November 1994 (1994-11), pages 10859-10863, XP001010361 ISSN: 0027-8424 cited in the application
- MARTIN R ET AL: "A MYELIN BASIC PROTEIN PEPTIDE IS RECOGNIZED BY CYTOTOXIC T CELLS IN THE CONTEXT OF FOUR HLA-DR TYPES ASSOCIATED WITH MULTIPLE SCLEROSIS" JOURNAL OF EXPERIMENTAL MEDICINE, TOKYO, JP, vol. 173, no. 1, January 1991 (1991-01), pages 19-24, XP000578296 ISSN: 0022-1007 cited in the application
- STINISSEN P ET AL: "Autoimmune pathogenesis of multiple sclerosis: role of autoreactive T lymphocytes and new immunotherapeutic strategies." CRITICAL REVIEWS IN IMMUNOLOGY. 1997, vol. 17, no. 1, 1997, pages 33-75, XP009045645 ISSN: 1040-8401
- ZHANG J ET AL: "MHC-restricted depletion of human myelin basic protein-reactive T cells by T cell vaccination", SCIENCE, AMERICAN ASSOCIATION FOR THE ADVANCEMENT OF SCIENCE, WASHINGTON, DC; US, vol. 261, no. 5127, 10 September 1993 (1993-09-10), pages 1451-1454, XP002091570, ISSN: 0036-8075

## Description

### BACKGROUND OF THE INVENTION

### 1. Field of the Invention

The present invention relates generally to the field of treatment of autoimmune disease, such as multiple sclerosis (MS). More particularly, it concerns a CD8⁺ T cell vaccine prepared by using immunogenic fragments of Myelin Basic Protein (MBP).

### 2. Description of Related Art

Multiple sclerosis (MS) is a demyelinating and chronic inflammatory disease of the central nervous system (CNS). The histopathologic hallmarks of the disease include focal infiltration of both CD4⁺ and CD8⁴ T cells together with other inflammatory cells in the white matter and demyelination with evidence of some axonal damage (Martin et al., Annu. Rev. Immunol. 1992; 1.0: 53; Keegan et al., Annu. Rev. Med. 2002; 53: 285). It has long been speculated that the T cell responses to certain myelin proteins, such as myelin basic protein (MBP), play an important role in the pathogenesis of MS. In experimental autoimmune encephalomyelitis (EAE), a classic animal model for MS, CD4⁺ T cells recognizing MBP have been found to induce CNS pathology characterized by extensive inflammation and mild demyelination (Zamvil et al., Nature 1985; 317: 355). Until recently it was not known that CD8⁺ T cells recognizing short peptides of MBP can induce EAE with distinct CNS pathology. These CD8⁺ T cells are cytotoxic toward target cells, recognize endogenously processed MBP and induced severe EAE upon adoptive transfer (Huseby et al., J. Exp. Med. 2001; 194: 669; Steinman, J. Exp. Med., 2001; 194: 27). It is important to note that CNS lesions induced by CD8⁺ cytotoxic T cells recognizing MBP in EAE are characterized by extensive demyelination, closely resembling MS pathology in humans (Huseby *et al.,* supra), suggesting that CD8⁺ cytotoxic T cells recognizing MBP are capable of causing injury of oligodendrocytes expressing both MHC class I molecules and MBP. These findings have raised new questions as to whether CD8⁺ cytotoxic MBP-reactive T cells play a similar role in MS.

In MS, there is some evidence indicating that the CD4⁺ T cell responses to MBP and other candidate myelin antigens may play an important role in the disease processes (Ota et al., Nature 1990; 346: 183; Martin et al., J. Exp. Med. 1991; 173: 19; Zhang et al., Ann. Neurol. 1992; 32: 330; Trotter et al., J. Neuroimmunol. 1998; 84: 172; Markovic-Plese et al., J. Immunol. 1995; 155: 982; Kerlero de Rosbo *et al.*, 1997; 27: 3059; Bieganowska et al., J. exp. Med. 1997; 185: 1585; Wallstrom et al., Eur. J. Immunol. 1998; 28: 3329; Lindert et al., Brain 1999; 122: 2089; Zhang et al., J. Exp. Med. 1994; 179: 973; Tejada-Simon et al., Intern. Immunol. 2000; 12: 1641). The results accumulated so far suggest that CD4⁺ MBP-reactive T cells undergo *in vivo* activation and clonal expansion in MS patients compared to healthy controls (Zhang et al., J. Exp. Med. 1994; 179: 973, Allegretta et al., Science 1990; 247: 718; Chou et al., J. Neurosci. Res. 1989; 23: 207; Vandervyver et al., Eur. J. Immunol. 1995; 25: 958; Wucherpfennig et al., J. Immunol. 1994; 152: 5581) and occur at an increased precursor frequency during acute exacerbation (Tejada-Simon et al., Intern. Immunol. 2000; 12: 1641). Compared to CD4⁺ MBP-reactive T cell counterparts, the potential involvement of CD8⁺ cytotoxic MBP-reactive T cells in the pathogenesis of MS is unknown. Tsuchida and co-workers reported the identification of CD8⁺ cytotoxic T cells in the blood of MS patients as well as in healthy individuals (Tsuchida et al., Proc. Natl. Acad. Sci: USA 1994; 91: 10859). Some of these CD8⁺ T cell lines appeared to recognize endogenously processed myelin peptides, suggesting their potential role in the injury of oligodendrocytes that constitutively express MHC class I molecules (but not class II molecules) and the myelin antigens. A subsequent study confirmed that HLA-A2 restricted CD8⁺ T cell lines recognizing the 110-118 peptide of MBP could mediate lysis of human oligodendrocytes (Jurewicz et al., J. Immunol. 1998; 160: 3056). However, it remains unknown whether CD8⁺ T cells recognizing MBP are sensitized *in vivo* to undergo activation and expansion in MS patients as compared to healthy controls and whether there are additional epitopes associated with other MHC class I molecules.

### SUMMARY OF THE INVENTION

The present invention is directed to the isolation of CD8⁺ cytotoxic T cells that recognize multiple sclerosis related antigens including but not limited to antigens such as Myelin Basic Protein (MBP), and/or fragments thereof. The MBP fragments may be peptides that comprise 8 or more amino acids of any of the sequences set forth in SEQ ID NOS: 1-4. The MS associated antigen may also be proteolipid protein (PLP) or myelin oligodendrocyte glycoprotein (MOG).

In one embodiment, the present invention employs fragments of MBP, which bind HLA-A2 and HLA-A24 receptors with high affinity. Among the fragments of MBP employed by embodiments of the present invention are those set out as SEQ ID NOS: 1-4. The fragments may also be homologs having conservative amino acids at one or more position of the fragment but which still bind to the HCA-A2 and HCA-A4 receptors. Other fragments of MBP include fragments comprising amino acids 83-99 or 151-170 of MBP.

In another embodiment, the present invention describes a method for preparing a vaccine useful in the treatment or prevention of MS comprising obtaining a population of peripheral blood mononuclear cells (PBMCs) comprising T cells from a patient to be treated; enriching said population for CD8⁺ T cells preferably by reducing or depleting the number of CD4⁺ cells in the population; and incubating said CD8⁺ T cell enriched population with one or more peptides corresponding to MBP-fragments capable of binding to HLA-A2 and HLA-A24 so as to increase the number of CD8⁺ T cell clones in the population specific for said MBP polypeptides. The population of CD8⁺ T cells specifically responsive to the MBP's may be further expanded by, for example, alternately stimulating said cells with the corresponding MBP peptides and a mitogen, for example, in the presence of antigen presenting cells (APCs).

Also described are methods of testing CD8⁺ T cell vaccines for their cytotoxicity against autologous cells primed with MBP-fragments, including but not limited to peptides having an amino acid sequence corresponding to SEQ ID NOS: 1,2,3 or 4.

A vaccine may be for use in a method of treating MS by administering to a patient in need of the treatment with autologous CD8⁺ T cells responsive to the MBP fragments and preferably capable of binding to HLA-A2 and HLA-A24.

In yet another aspect, the present disclosure provides a method for producing an autologous CD8⁺ T cell vaccine by means of isolating or generating CD 8⁺ T cells that have cytotoxic activity against MPB-reactive CD8⁺ T cells of the patient. Under these methods, an autologus T cell memory clones are selected for their reactivity against MBP-reactive CD8⁺ T cells of the patient.

### BRIEF DESCRIPTION OF THE DRAWINGS

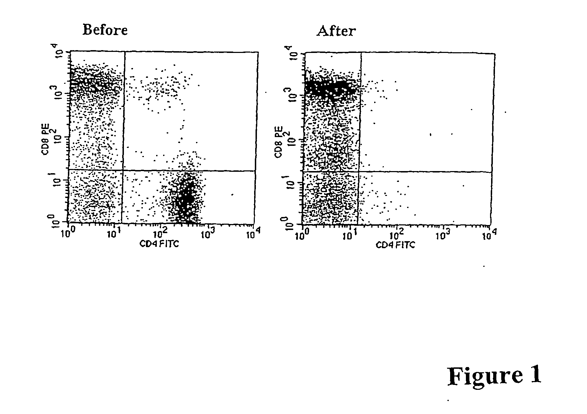

**Figure 1A** and **1B** illustrates percentage of CD4⁺ T cells and CD8⁺ T cells before and after T cell depletion. PBMC derived from an MS patient (MS-4) were analyzed for percentage of CD4⁺ T cells and **CD8⁺** T cells before (left panel) and after (right panel) magnetic bead-depletion of CD4⁺ T cells. The rate of CD4⁺ T cell depletion was always greater than 98% in all 30 experiments. The average percentage of CD8⁺ T cells in PBMC depleted for CD4⁺ T cells was 72 ± 8%.

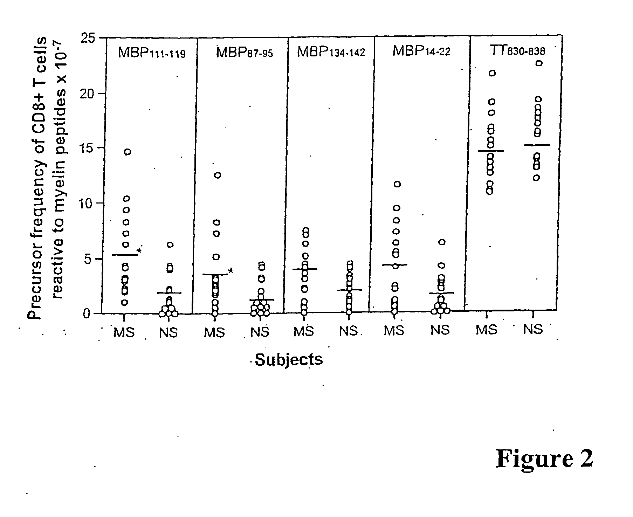

**Figure 2** shows the estimated precursor frequency of CD8⁺ T cells reactive to MBP-derived peptides in patients with MS and normal subjects (NS). The CD8⁴ T cell frequency analysis was performed by the split-well method in which responder PBMC fractions pre-depleted for CD4⁺ T cells were cultured with irradiated autologous PBMC that were not fractionated in the presence of the indicated MBP-derived peptides, respectively. A synthetic peptide corresponding to an immunodominant epitope of tetanus toxoid (residues 830-838) was used as a control. Each open circle represents the frequency of CD8⁺ T cells in each individual. The data are expressed as the estimated frequency of CD8⁺ T cells recognizing the MBP-derived peptides in CD4-depleted fractions of PBMC.

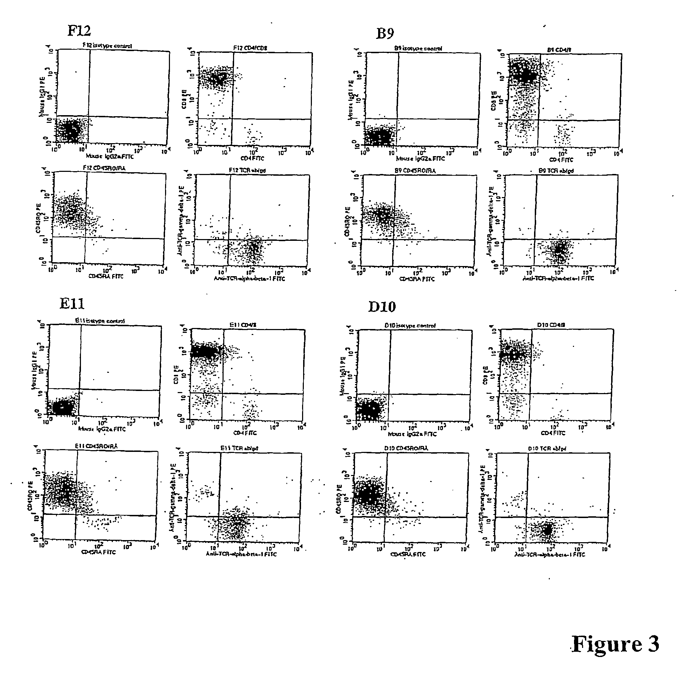

**Figure 3** shows phenotypic expression of CD8⁺ T cells reactive to MBP-derived peptides. CD8⁺ T cell lines (E11, D10, B9 and F12) were analyzed for the phenotypic expression with a panel of monoclonal antibodies to TCRαβ/TCRγδ, CD4/CD8, CD45RA/CD45RO.

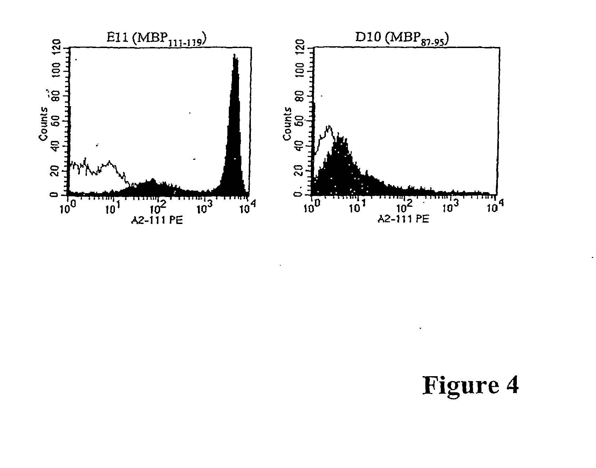

**Figure 4** shows analysis of MHC Class I tetramer for binding to cloned CD8⁺ T cell lines by flow cytometry. Two A2-restricted CD8⁺ T cell lines that recognized MBP₁₁₁₋₁₁₉ peptide (E11) and MBP₈₇₋₉₅ peptide (D 10) were analyzed by flow cytometry using an HLA-A2- MBP_{111- 119} tetramer. The open profiles represent staining of T cells with a PE-conjugated control antibody. The solid profiles indicate staining of T cells with the tetramer in the same representative experiment.

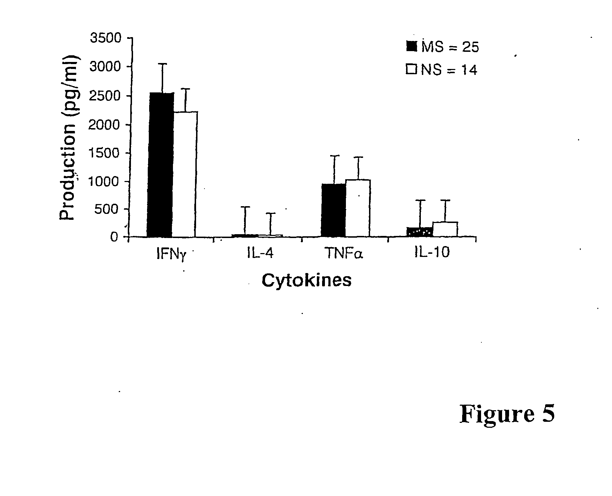

**Figure 5** illustrates the cytokine profile of CD8⁺ T cells recognizing MBP-derived peptides. Cytokine production of the resulting CD8⁺ MBP-reactive T cell lines derived from patients with MS (MS, n=25) and from normal subjects (NS, n=14) was measured by ELISA. The T cell lines were challenged with the corresponding peptide, respectively, and the supernatants were tested after 48 hours for concentrations of the indicated cytokines. The bars indicate the mean concentration (pg/ml) ± SEM. The detection limit of the assays for all cytokines was less than 25 pg/ml.

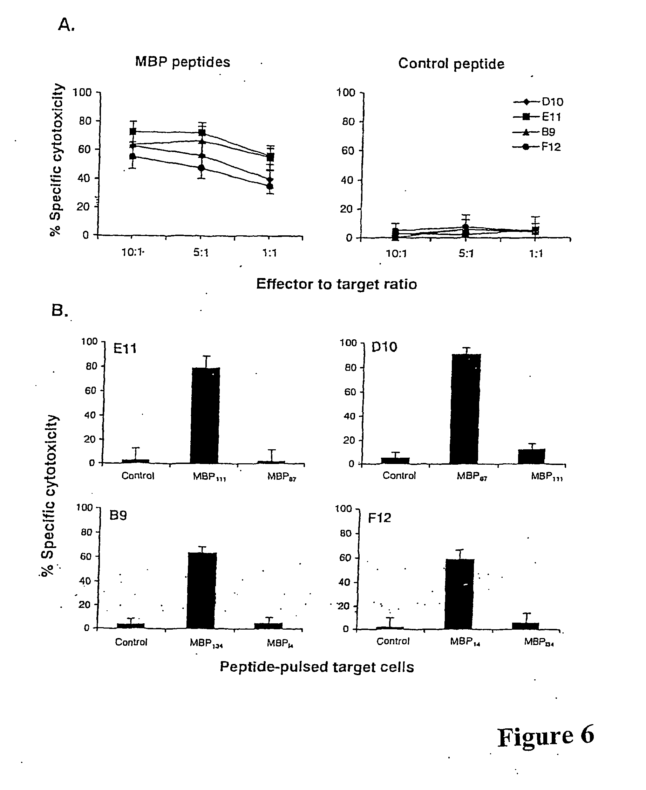

**Figure 6A and 6B** shows cytotoxic activity of CD8⁺ T cell lines recognizing MBP-derived peptides against autologous target cells. Four representative CD8⁺ T cell lines reactive to MBP-derived peptides, E11 for MBP₁₁₁₋₁₁₉, D10 for MBP₈₇₋₉₅, B9 for MBP₁₃₄₋₁₄₂ and F12 for MBP₁₄₋₂₂ were examined for cytotoxicity in LDH-release assays. *Panel A*. CD8⁺ T cell lines were tested for cytotoxic activity against autologous target cells pulsed with corresponding peptides at the indicated effector (CD8⁺ T cells) to target (autologous EBV-transformed B cells) ratio. A synthetic 9-mer peptide corresponding to a unrelated TCR CDR3 sequence (STRQGPQET) (SEQ ID NO: 5) was used as a control. *Panel B*. The same CD8⁺ T cell lines were analyzed for cytotoxicity against autologous target cells pulsed with different peptides of MBP. The same autologous target cells pulsed with the irrelevant TCR peptide served as a control peptide. The effector to target ratio was 10.

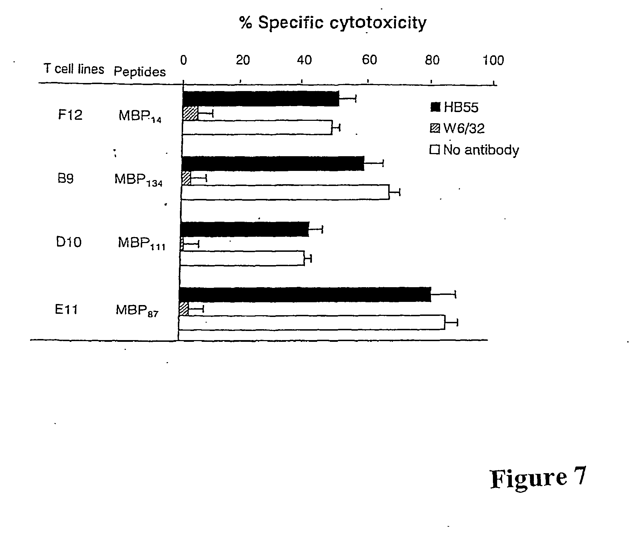

**Figure 7** illustrates MHC restriction of CD8⁺ cytotoxic T cell lines. The selected CD8⁺ cytotoxic T cell lines recognizing MBP-derived peptides were tested for specific cytotoxicity against autologous target cells in the presence and absence of two monoclonal antibodies to MHC class I (W6/32) and class II (HB55) used at a concentration of 20 µg/ml. The effector to target ratio was 10 for all experiments. Data are expressed as % specific cytotoxicity. The procedure used is the same as that described in the Figure 5 legend.

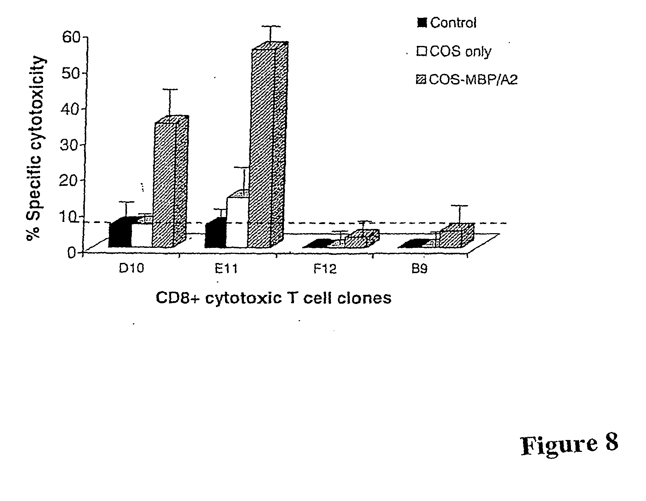

**Figure 8** shows cytotoxic activity of CD8⁺ T cell lines reactive to MBP-derived peptides against COS cells transfected with human MBP and HLA-A2 genes.

The selected CD8⁺ cytotoxic T cell lines were tested for cytotoxic activity in LDH-release assays using COS cells transfected with human MBP and HLA-A2 genes. The effector to target ratio was 10. Non-transfected COS cells were used as a control.

### DETAILED DESCRIPTION

Before the present compounds, products and compositions and methods are disclosed and described, it is to be understood that, as used in the specification and the appended claims, the singular forms "a," "an" and "the" include plural referents unless the context clearly dictates otherwise.

Autoreactive T cells of CD4 and CD8 subsets recognizing myelin basic protein (MBP) contribute in the pathogenesis of multiple sclerosis (MS). Unlike CD4⁺ MBP-reactive T cells that induce extensive CNS inflammation and mild demyelination in EAE, CD8⁺ cytotoxic T cells recognizing MBP-derived peptides directly contribute to severe CNS demyelination in EAE presumably through induction of injury of oligodendrocytes (Huseby et al., J. Exp. Med. 2001; 194:669). The distinct role of these CD8⁺ cytotoxic T cells is of particular relevance to MS where demyelination represents the most significant CNS pathology associated with neurologic deficits. There is evidence in the literature on the CD4⁺ T cell responses to candidate myelin antigens in MS and the preliminary therapeutic attempts to suppress or eliminate CD4⁺ myelin-reactive T cells (Zhang et al., Science 1993; 261: 1451; Vandenbark et al., Nat. Med. 1996; 10: 1109). In contrast, the functional properties and the potential role of CD8⁺ T cells in recognizing myelin antigens in MS are virtually unknown. Part of the reason for lack of advances in this area is related to technical difficulties in detection and generation of CD8⁺ T cells reactive to myelin antigens. This disclosure discloses an approach for identifying CD8⁺ T cells that are reactive to MS associated antigens, preferably MBP and/or fragments thereof and the effective generation of CD8⁺ T cell lines, which are useful in the treatment of MS, in monitoring the progression of the disease and the monitoring of therapeutic response to treating of the disease. The methods of the present disclosure are also useful for the diagnosis and monitoring of the progression of MS.

The approach includes obtaining PBMCs from an MS patient in need of treatment; pre-depleting the population of PBMCs of CD4⁺ T cells resulting in a population of PBMCs enriched for CD8⁺ cells with MS associated antigens so as to increase the number of CD8⁺ T cells in the population and optionally repeating the stimulation cycle in the presence or absence of antigen presenting cells. The production of T cell lines using alternating cycles of stimulation is described in U.S. Patent Application No. 09/952,532 and International Application Nos. PCT/US02/02887 (Published as WO 03/024393) and PCT/US03/24548 (Published as WO 04/15070).

Methods of selecting CD8⁺ T cell lines with reactivity to particular antigens, including immunogenic fragments of MBP are also included. In this context, immunogenic means the ability to induce or sustain a T cell response including, but not limited to, a proliferative response, or for example, to stimulate the production of cotoxins by T cells. The methods of identifying MBP immunogenic fragments are also disclosed and amino acid sequences for four MBP immunogenic fragments with high binding affinity to HLA-A2 and HLA-A24 are provided.

The data described herein provides important evidence that CD8⁺ cytotoxic T cells recognizing MBP-derived peptides are involved in the pathogenesis of MS and therefore indicates the need for development of treatments that would reduce the number of these CD8⁺ cytotoxic T cells in MS patients.

The estimated frequency of CD8⁺ cytotoxic T cells recognizing the identified MHC class I peptides of MBP is in the range of 3.4 to 5.4 x 10⁻⁷ in PBMC derived from MS patients and 1.1 - 2.0 x10⁻⁷ in the control group. There are several issues related to this finding. First, the observed frequency of CD8⁺ cytotoxic T cells recognizing the identified regions of MBP in PBMC is relatively lower than that of CD4⁺ T cells recognizing immunodominant peptides of MBP in MS patients (1-2x10⁻⁶ in PBMC) under the same experimental condition (Ota et al., Nature 1990; 346: 183; Zhang et al., J. Exp. Med. 1994; 179: 973; Tejada-Simon et al., Inern. Immunol. 2000; 12: 1641). Like CD4⁺ MBP-reactive T cells, these CD8⁴ cytotoxic MBP-reactive T cells can also be detected in healthy individuals (Ota et al., Nature 1990; 346: 183; Martin et al., J. Exp. Med. 173: 19; Zhang et al., J. Exp. Med. 1994; 179: 973; Tejada-Simon et al., Inern. Immunol. 2000; 12: 1641). However, the estimated T cell frequency is significantly. higher in MS patients than that in controls. The differences appear to be more significant than those for CD4⁺ MBP-reactive T cells seen in MS patients and controls. It should also be noted that unlike CD4⁺ MBP-reactive T cells that are naive T cells expressing both CD45RA and CD45RO (Muraro et al., J. Immunol. 2000; 164: 5474), these CD8⁺ cytotoxic T cells identified here belong to antigen-experienced memory T cell subset expressing CD45RO but not CD45RA phenotype. Secondly, the finding suggests that these CD8⁺ cytotoxic T cells recognizing MBP-derived peptides may undergo in vivo activation in MS patients.

In this regard, there is increasing evidence indicating that MBP-reactive T cells can be activated by a variety of microbial antigens through the mechanism known as molecular mimicry (Oldstone, Curr. Topics Microbiol. Immunol. 1989; 145: 127; Oldstone, FASEB J. 1998; 12: 1255; Hafler, J. Clin. Invest. 1999; 104: 527; Tejada-Simon et al., Annals of Neurology 2003; 53: 189). Recently, we identified sequence homology between HHV-6, a suspected etiologic agent for MS, and MBP and demonstrated that CD4⁺ T cells cross-reactive with both antigens are sensitized in MS patients as opposed to healthy individuals (Tejada-Simon et al., Annals of Neurology 2003; 53: 189). Although the regions of MBP identified here do not share complete sequence homology with myelin proteins, it is established that TCR degeneracy occurs in MBP-reactive T cells, which renders them able to recognize microbial antigenic peptides of incomplete sequence match as long as the TCR contact residues required for T cell recognition are preserved (Wucherpfennig et al., Cell 1995; 80: 695; Hemmer et al., J. Exp. Med. 1997; 185:1651; Kozovska et al., Eur. J. Immunol. 1998; 28: 1894). Finally, it is arguable that although the cell culture-based split-well method has been proven useful in comparing the T cell frequency between.individual samples when used consistently (Ota et al., Nature 1990; 346: 183; Zhang et al., J. Exp. Med. 1994; 179: 973; Tejada-Simon et al., Intern. Immunol. 2000; 12: 1641; Zhang et al., Science 1993; 261: 1451; Tejada-Simon et al., J. Virol. 2002; 76: 6147), the actual precursor frequency of CD8⁺ cytotoxic T cells may be under-estimated using the method. A number of studies on the precursor frequency analysis of CD4⁺ specific T cells have provided some indications. It has been reported that the frequency of CD4⁺ MBP-reactive T cells in MS is in the range of 4 x10⁻⁵ by ELISPOT based on ex vivo secretion of cytokines in response to antigenic stimulation, which is higher than that measured in 1-2 x10⁻⁶ by the split-well method employed here. In this study, however, ELISPOT is not applicable to quantitative detection of CD8⁺. T cells because the source of ex vivo secretion of γ-IFN could not be distinguished between CD8⁺ T cells and CD4⁺ T cells, even though they were irradiated. In this study, further characterization has confirmed that these CD8⁺ T cells recognizing MBP-derived peptides are cytotoxic in nature. They recognize and are cytotoxic toward both autologous target cells pulsed with the MBP peptides and endogenously processed MBP in the context of MHC class I molecules as evidenced in a series of experiments involving COS cells doubly transfected with HLA-A2 and human MBP genes. This finding is of particular importance in view of a potential role of CD8⁺ cytotoxic T cells in the injury of oligodendrocytes that express both class I molecules and MBP. The findings are in agreement with an earlier study by Jurewicz and colleagues who reported the specific cytotoxicity of CD8⁺ MBP₁₁₀₋₁₁₈ reactive T cells toward A2⁺ human oligodendrocytes (Jurewicz et al., J. Immunol. 1998; 160: 3056). The CD8⁺ cytotoxic T cells reactive to MBP-derived peptides as described here are reminiscent of CD8⁺ T cells of similar functional properties in EAE, which are able to induce extensive CNS demyelination potentially through specific recognition and cytotoxic activity toward oligodendrocytes (Huseby et al., J. Exp. Med. 2001; 194: 669).

The following examples are included to demonstrate preferred embodiments of the invention. Is should be appreciated by those of skill in the art that the techniques disclosed in the examples which follow represent techniques disclosed by the inventor to function well in the practice of the invention, and thus can be considered to constitute preferred modes for its practice. However, those of skill in the art should, in light of the present disclosure, appreciate that many changes can be made in the specific embodiments which are disclosed.

The present invention has multiple aspects, illustrated by the following non-limiting examples.

### EXAMPLES

### Example 1

### Identification of MBP fragments with high binding affinity to HLA-A2 and HLA-A24 receptors.

TEPITOPE, (Vaccinome website) an application that allows the identification of HLA class I ligand binding epitopes (Schroers et al., Cancer Res. 2002; 62: 2600; Engelhard Annu. Rev. Immunol. 1994; 12:181; Manici et al., J. Exp. Med. 1999; 189: 871), was used to screen amino acid sequence of human myelin basic protein (MBP) for fragments capable of binding to HLA-A2 and HLA-A24. Two fragments with predicted HLA-A2 binding sequences (1% threshold) and two fragments with predicted HLA-A24 binding sequences (1% threshold) were identified. The amino acid sequences of the fragments are as follows:

| **Amino acid Sequence of the Fragment** | **Corresponding SEQ ID NO.** | **Corresponding Amino acids of Human BMP** | **Identified for its binding to** |
|---|---|---|---|
| MIBPVVBFFKNIV | SEQ ID NO. 1 | 87-95 | HLA-A2 |
| SLSRFSWGA | SEQ ID NO. 2 | 111-119 | HLA-A2 |
| DYKSAHKGF | SEQ ID NO. 3 | 134-142 | HLA-A24 |
| KYLATASTM | SEQ ID NO. 4 | 14-22 | HLA-A24 |

Peptides with SEQ ID NOS: 1-4 were then synthesized using the Mayfield method and were purified using HPLC (MD Anderson Cancer Center Peptide Core, Houston, TX). The purity of the peptides was greater than 90%.

### Example 2

### Precursor frequency analysis of CD8⁺ T cells recognizing MBP-derived peptides in MS patients and healthy controls

Fifteen patients with relapsing-remitting or secondary progressive MS (Poser et al., Ann. Neurol., 13(3):227-31, 1983) were included in the study. Patients had not been treated with immunosuppressive or immunomodulatory drugs (*e.g.*, Azathioprine, Cyclophosphamide, Beta Interferons or Glatiramer Acetate) at least 3 months before entering the study. The protocol was approved by the Institutional Review Board at Baylor College of Medicine. A group of 15 healthy subjects matched for age and sex with the MS group was included as controls. The clinical characteristic/demographic data and HLA-A2 and -A24 genotypes of MS patients and control subjects are shown in Table 1.

The precursor frequency of T cells recognizing the selected peptides of MBP with SEQ ID NOS: 1-4 was estimated in MS patients and controls using the split-well method (Ota et al., Nature 1990; 346: 183; Zhang et al., J. Exp. Med. 1994; 179: 973). The initial attempts to detect CD8⁺ T cell responses to the MBP peptides in unfractionated peripheral blood mononuclear cells yielded low frequencies of specific T cell isolates of mixed CD4⁺ and CD8⁺ phenotypes. The approach to detecting CD8⁺ T cells reactive to MBP-derived peptides was improved subsequently by pre-depleting CD4⁺ T cells. The depletion was achieved by using a method described below.

Peripheral blood mononuclear cells (PBMC) were isolated from the peripheral blood of MS patients and healthy individuals by Ficoll separation. CD4⁺ T cells were pre-depleted using magnetic beads coupled with an anti-CD4 antibody (Dynal ASA, Oslo, Norway). Briefly, PBMC were incubated with magnetic beads coated with the antibody at a bead to cell ratio of 10 for 30 min with gentle shaking. Unbound PBMC fractions were collected by magnetic separation. The depletion rate for CD4⁺ T cells was greater than 98% in all cases. The resulting CD4-depleted fractions typically contained 72 ±8% CD8⁺ T cells as determined by flow cytometric analysis. A representative experiment is shown in Figure 1.

The resulting PBMC fractions were then seeded in 96-well U-bottomed microtiter plates at a density of 50,000 cells/well together with 10⁵ of autologous unfractionated PBMC that were irradiated to provide "helper" function of CD4⁺ T cells while they themselves were unable to proliferate in response to the antigens. Peptides with SEQ ID NOS: 1 -4. were added at a final concentration of 20 µg/ml to cultures. A total of 32 wells were set for each peptide. A synthetic peptide corresponding to an immunodominant peptide of tetanus toxoid (residues 830-838) was included in the precursor frequency analysis as a negative control. Cells were cultured at 37°C in 5% CO₂ atmosphere. After 7 days, all cultures were tested for specific proliferation to the corresponding peptides by tritiated thymidine incorporation. In brief, each well was split into four aliquots (approximately 10⁴ cells per aliquot) and cultured in duplicate with 10⁵ autologous PBMC in the presence and the absence of the corresponding MBP-derived peptides at 20 µg/ml. Cultures were kept for three days and pulsed with [³H]-thymidine (Nycomed Amersham, Arlington Heights, IL) at 1 µCi per well during the last 16 hours of culture. Cells were then harvested using an automated cell harvester and [³H]-thymidine incorporation was measured in a betaplate counter (Wallac, Turku, Finland).

A well/culture was defined as specific for the peptide when the CPM were greater than 1,500 and exceeded the reference CPM (in the absence of the peptide) by at least three times. The frequency of specific CD8⁺ T cells was then estimated by dividing the number of positive wells by the total number of CD4-depleted PBMC seeded in the initial culture.

As shown in Figure 2, the results revealed that the average precursor frequency of CD8⁺ T cells recognizing MBP-derived peptides was estimated in the range of 3.4 to 5.4X10⁻⁷ in CD4⁺ T cell-depleted PBMC obtained from patients with MS, which was considerably higher than that in control subjects (1.1 to 2.1X10⁻⁷), especially for MBP₁₁₁₋₁₁₉ and MBP₈₇₋₉₅ (p<0.05). In contrast, the frequency of CD8⁺ T cells recognizing an immunodominant epitope (residues 830-838) of tetanus toxoid, a recall antigen, did not differ significantly between MS patients and healthy controls (Figure 2).

### Example 3

### Phenotypic analysis of generated CD8⁺ T cell lines

A panel of 39 CD8⁺ T cell lines generated in Example 2 were characterized for phenotypic expression, cytokine profile and specific cytotoxic activity toward autologous target cells. The panel included 25 T cell lines from MS patients and 14 T cell lines from healthy controls and was representative for reactivity to all four MBP-derived peptides (Table 2).

To analyze the phenotypic expression, 10⁵ cells of each T cell line were washed in PBS containing 1% FBS and 0.1% sodium azide (FBS-PBS) and re-suspended in 100 µl FBS-PBS containing a 1:100 dilution of fluorochrome-labeled antibody (Simultest CD4/CD8, CD45RA/CD45RO, TCRα/β/TCRγ/δ, Becton Dikinson Immunocytometry Systems, San Jose, CA) or appropriate Ig isotype controls (γ2a-FITC/γ1-PE, Becton Dickinson Immunocytometry Systems). After incubation for 30 min on ice, the cells were washed three times in FBS-PBS, and fixed in 1% formaldehyde for flow cytometric analysis.

It was found that the selected CD8⁺ T cell lines express TCRαβ/CD8 (>95% on average) but not CD4 (<5%) and CD45RO but not CD45RA, regardless of their reactivity to the various MBP-derived peptides. The findings indicate that the selected T cell lines belong to the CD8⁺ memory T cell subset.

### Example 4

### Cytokine production of CD8⁺ T cell lines

The cytokine profile of the resulting CD8⁺ T cell lines was analyzed to determine whether they belonged to a Th1 or a Th2 subset. The selected T cell lines (n=39) were first challenged with autologous APC pulsed with the corresponding MBP peptides. The cytokine profile was determined quantitatively using ELISA kits (PharMingen, San Diego, CA). Microtiter plates (96-wells, NUNC Maxisorp) were coated overnight at 4°C with 1 µg/well of a purified mouse capturing monoclonal antibody to human cytokine (IL-4, IL-10, TNF-α, γ-IFN) (PharMingen). Plates were washed and non-specific binding sites were saturated with 10 % (w/v) fetal bovine serum (FBS) for 1 hour and subsequently washed. Supernatants and cytokine standards were diluted with PBS and added in duplicate wells. Plates were incubated at 37°C for 2 hr and subsequently washed with PBS-T. Matched biotinylated detecting antibody was added to each well and incubated at room temperature for 2 hours. After washing, avidin-conjugated horseradish peroxidase was added and plates were incubated for 1 hour. 3,3',5,5'-tetramethylbenzidine (TMB, Sigma) was used as a substrate for color development. Optical density was measured at 450 nm using an ELISA reader (Bio-Rad Laboratories, Hercules, CA) and cytokine concentrations were quantitated by Microplate computer software (Bio-Rad) using a double eight-point standard curve.

As seen in Figure 5, the selected CD8⁺ T cell lines recognizing the MBP-derived peptides predominantly produced TNF-α and IFN-γ but not IL-4 and IL-10, thus belonging to a Th1 phenotype. No significant quantitative differences between the MS-derived T cell lines and the T cell lines derived from the control subjects could be discerned.

### Example 5

### Establishing clones from the representative T cell lines

Four representative T cell lines (E11, D10, B9 and F12) were selected for their recognition of the four MBP peptides and further cloned by limiting dilution. Briefly, T cells were plated out at one cell per well in 96-well U-bottomed plates under limiting dilution conditions in the presence of irradiated PBMC (100,000 cells per well) and phytohemaglutinin-protein (PHA-P) at 2 µg/ml. Cells were cultured in IL-2 containing medium for 10-12 days with medium change every 3 to 4 days. Growth positive wells were confirmed for the phenotypic expression of CD8 and for reactivity to the corresponding peptides. The obtained T cell clones were further expanded by alternate stimulation with the corresponding MBP peptides and PHA-P. in the presence of antologous APC.

Two such cloned CD8⁺ T cell lines recognized MBP₁₁₁₋₁₁₉ peptide (E11) (SEQ ID NO: 2) and MBP₈₇₋₉₅ peptide (D10) (SEQ ID NO: 1), respectively, in the context of HLA-A2 while two other CD8⁺ T cell lines were A24 restricted and reacted with MBP₁₃₄₋₁₄₂ peptide (B9) (SEQ ID NO: 3) and MBP₁₄₋₂₂ peptide (F12) (SEQ ID NO: 4). Figure 3 illustrates the representative phenotypic expression of four cloned T cell lines described above.

Specific binding of an HLA-A2 tetramer to selected CD8⁺ T cell lines was then examined. HLA-A2-MBP₁₁₁₋₁₁₉ tetramer was obtained from Immunomics (San Diego, CA). As shown in Figure 4, the HLA-A2/MBP₁₁₁₋₁₁₉ tetramer exhibited greater than 90% specific binding to a CD8⁺ T cell line (E11) recognizing peptide MBP₁₁₁₋₁₁₉ but not to an A2⁺ CD8⁺ T cell line (D10) recognizing peptide MBP₈₇₋₉₅.

### Example 6

### Cytotoxicity of MBP-reactive CD8⁺ T cell clones against autologous cells

All 39 selected CD8⁺ T cell lines were analyzed for cytotoxic activity toward autologous target cells. For this purpose, a panel of autologous B cell lines was generated from patients and controls using EBV transformation on a procedure described previously (Zhang et al., J. Neuroimmunol. 1989; 23: 249; Tejada-Simon et al., Immunology 2002; 107:403). The generated cell lines were pulsed with corresponding peptides of BMP and used as autologous target cells. Pulsing of B cells was carried out by incubating cells with MBP-derived peptides or a control T cell receptor peptide (40 µg/ml), respectively, for 2 hrs followed by washing to remove free peptides.

Cytotoxicity test was performed using a lactate dehydrogenase (LDH)-release assay (Promega Madison, WI). LDH release was measured in an enzymatic assay according to manufacturer's instruction. Briefly, CD8⁺ T cells (50,000 effector cells/well) were incubated with autologous cells at an effector to target ratio of 10 and centrifuged once at 250 x g. Unpulsed autologous B cells and non-transfectants were used as controls. RPMI1640 without phenol red was used throughout the assay to avoid background absorbance. After incubation at 37°C and 5% CO₂ for 4 hr, the plates were centrifuged again. 50 µl of supernatant was transferred to another plate and mixed with Substrate Mix provided in the test kits. The reaction was stopped after 30 min and read at 490 mm absorbance_{.} Specific cytotoxicity was calculated as: %cytotoxicity = (experimental release - spontaneous release) / (maximum release - spontaneous release) x 100.

The results revealed that 21/25 (84%) of MS-derived and 11/15 (73%) control-derived CD8⁺ T cell lines exhibited a specific cytotoxic effect, as defined by specific cytolysis greater than 30%, on autologous target cells pulsed with the corresponding peptide but not on the same autologous target cells that were either unpulsed or pulsed with an irrelevant peptide. However, no significant differences in percentage of specific cytolysis between MS-derived and control CD8⁺ T cell lines could be discerned. Representative experiments with four selected T cell lines derived from three MS patients are shown in Figure 6.

Furthermore, the observed cytotoxic effect was restricted by MHC class I molecules as the cytotoxicity could be inhibited by the addition of a monoclonal antibody (W6/32) to MHC class I molecules while an antibody (HB55) to MHC class II molecules had no effect (Figure 7). For these MHC restriction experiments, purified monoclonal antibodies to MHC class I (W6/32) or MHC class II (HB55) were added at (20 µg/ml) during incubation of effector cells with target cells in cytotoxicity assays described above.

### Example 7

### Cytotoxicity of MBP-reactive CD8⁺ T cell clones against cells expressing processed MBP in combination with MHC class I molecules

It was important to identify whether selected CD8⁺ T cells have cytotoxic effect on oligodendrocytes that express both MHC class I molecules and endogenously processed MBP. To address this question, the following test was developed. Under the test, COS cells were transfected with HLA-A2*01 gene and human MBP gene. Specifically, cDNA encoding human MBP and human HLA-A2 were constructed into pBud CE4.1 vector that contained two promoters (P_{CMV} promoter and P_{EF-α1} promoter). The recombinant DNA was transfected into COS-7 cells using LipofectAMINE 2000 (Invitrogen, San Diego, CA). The stable transfectants were selected using selective medium containing Zeocin at 400 µg/ml (Invitrogen, San Diego, CA). Stable expression of MBP and HLA-A2 were evaluated by incubating the cells with conjugated monoclonal antibodies to MBP (Sigma, St. Louise, MO) or HLA-A2 (BD Pharmingen, San Diego, CA) and analyzed subsequently by flow cytometry.

Selected MS-derived CD8⁺T cell lines that were cytotoxic toward peptide-pulsed autologous target cells were analyzed in cytotoxicity experiments using transfected COS cells. The two HLA-A2⁺ CD8⁺ T cell lines (E11 and D10) displayed specific cytotoxicity toward A2-MBP transfected COS cells but not non-transfectants while the other CD8⁺ cytotoxic T cell lines of A24- restriction (B9 and F12) had no cytotoxic effect on transfected COS cells. Results on four representative CD8⁺ T cell lines are shown in Figure 8, indicating specific recognition and cytotoxicity towards target cells expressing both human MBP and HLA-A2.

**Table 1. Clinical characteristics and HLA-A2 and A-24 genotypes of MS patients and healthy individuals**

| Subject | Age | Sex | RR/SP | EDSS | Duration (yrs.) | A*0201/A*2402 |
|---|---|---|---|---|---|---|
| MS-1 | 36 | F | RR | 0 | 1 | ⁺ / ⁺ |
| MS-2 | 33 | F | RR | 1.0 | 7 | ⁺ / ⁺ |
| MS-3 | 55 | F | RR | 6.5 | 9 | - / - |
| MS-4 | 41 | F | RR | 3.5 | 8 | ⁺ / - |
| MS-5 | 46 | F | RR | 2.0 | 11 | ND |
| MS-6 | 47 | F | RR | 7.5 | 16 | - / - |
| MS-7 | 51 | F | SP | 6.0 | 14 | - / - |
| MS-8 | 54 | M | RR | 2.5 | 8 | ⁺ / - |
| MS-9 | 56 | M | RR | 6.0 | 24 | ⁺ / - |
| MS-10 | 46 | F | RR | 2.0 | 8 | ⁺ / ⁺ |
| MS-11 | 53 | F | RR | 3.5 | 13 | ⁺ / - |
| MS-12 | 54 | M | RR | 3.0 | 19 | - / ⁺ |
| MS-13 | 44 | M | RR | 3.0 | 6 | ⁺ / ⁺ |
| MS-14 | 47 | F | SP | 4.0 | 9 | - / - |
| MS-15 | 63 | F | SP | 6.5 | 17 | - / ⁺ |
| NS-1 | 46 | M | - | - | - | ⁺/⁺ |
| NS-2 | 34 | F | - | - | - | - / ⁺ |
| NS-3 | 42 | F | - | - | - | - / ⁺ |
| NS-4 | 34 | F | - | - | - | ⁺ / ⁺ |
| NS-5 | 21 | M | - | - | - | ⁺ / - |
| NS-6 | 22 | F | - | - | - | ⁺ / - |
| NS-7 | 43 | F | - | - | - | ND |
| NS-8 | 40 | F | - | - | - | ⁺ / - |
| NS-9 | 26 | M | - | - | - | ⁺ / ⁺ |
| NS-10 | 45 | F | - | - | - | ⁺ / ⁺ |
| NS-11 | 30 | F | - | - | - | ⁺ / - |
| NS-12 | 46 | F | - | - | - | - / - |
| NS-13 | 37 | M | - | - | - | ⁺/⁺ |
| NS-14 | 35 | F | - | - | - | -/- |
| NS-15 | 35 | F | - | - | - | -/⁺ |

RR, relapsing-remitting MS; SP, secondary progressive MS; EDSS, expanded disability scale score. HLA typing was determined by RT-PCR using specific primers for A*0201 and A*2402.

**Table 2. Characteristics of selected CD8⁺ T cell lines for further characterization**

| Total # of T cell lines | Derived from (# of subjects) | Peptide specificity (# of lines) |
|---|---|---|
| 25 | MS patients (12) | Peptide 111-119 (9) |
| | | Peptide 87-95 (7) |
| | | Peptide 134-142 (5) |
| | | Peptide 14-22 (4) |
| 14 | Controls (8) | Peptide 111-119 (3) |
| | | Peptide 87-95 (4) |
| | | Peptide 134-142 (4) |
| | | Peptide 14-22 (3) |

## Claims

1. A method of making an autologous T cell vaccine for the treatment of multiple sclerosis comprising:
(a) providing a population of peripheral blood mononuclear cells comprising T cells obtained from a patient to be treated with the vaccine;
(b) enriching said population for CD8⁺ T cells; and
(c) adding fragments of multiple sclerosis (MS) associated antigens and optionally antigen presenting cells, wherein the MS associated antigens are selected from the group consisting of myelin basic protein (MBP), proteolipid protein (PLP), and myelin oligodendrocyte glycoprotein (MOG); and
(d) repeating steps (c) one or more times.

2. The method of claim 1 wherein one or more of said MS associated antigen fragments comprises a sequence set forth in any one of SEQ ID NOS:1-4.

3. The method of claims 1 or 2, wherein one or more of said MS associated antigen fragments comprises amino acids 83-99 or 151-170 of MBP.

4. The method of any one of claims 1 to 3 wherein step (c) further comprises adding IL-2.

5. The method of any one of claims 1 to 4 wherein step (c) further comprises adding a mitrogen.

6. The method of claim 5 wherein said mitogen is selected from the group consisting of phytohemagglutinin, conconavalin A , pokeweed mitogen, and monoclonal antibodies to CD3.

## Patentansprüche

1. Verfahren zur Herstellung eines autologen T-Zell-Impfstoffs zur Behandlung von multipler Sklerose, das Folgendes umfasst:
(a) das Bereitstellen einer Population von mononukleären Zellen des peripheren Blutes, die T-Zellen umfassen, die von einem mit dem Impfstoff zu behandelnden Patienten erhalten wurden;
(b) das Anreichern der Population mit CD8⁺-T-Zellen;
(c) das Hinzufügen von Fragmenten von mit multipler Sklerose (MS) assoziierten Antigenen und gegebenenfalls antigenpräsentierenden Zellen, worin die mit MS assoziierten Antigene aus der aus Myelin-Basischem Protein (MBP), Proteolipidprotein (PLP) und Myelin-Oligodendrozyten-Glykoprotein (MOG) bestehenden Gruppe ausgewählt sind; und
(d) das ein- oder mehrmalige Wiederholen von Schritt (c).

2. Verfahren nach Anspruch 1, worin eines oder mehrere der mit MS assoziierten Antigenfragmente eine in einer der Seq.-ID Nr. 1 bis 4 dargelegte Sequenz umfassen.

3. Verfahren nach Anspruch 1 oder 2, worin eines oder mehrere der mit MS assoziierten Antigenfragmente die Aminosäuren 83 bis 99 oder 151 bis 170 von MBP umfassen.

4. Verfahren nach einem der Ansprüche 1 bis 3, worin Schritt (c) weiters das Hinzufügen von IL-2 umfasst.

5. Verfahren nach einem der Ansprüche 1 bis 4, worin Schritt (c) weiters das Hinzufügen eines Mitogens umfasst.

6. Verfahren nach Anspruch 5, worin das Mitogen aus der aus Phytohämagglutinin, Conconavalin A, Kermesbeerenmitogen und monoklonalen Antikörpern gegen CD3 bestehenden Gruppe ausgewählt ist.

## Revendications

1. Méthode de préparation d'un vaccin de cellules T autologue pour le traitement de la sclérose en plaques, comprenant :
(a) réaliser une population de cellules mononucléaires de sang périphérique comprenant des cellules T obtenues d'un patient à traiter avec le vaccin ;
(b) enrichir ladite population pour des cellules T CD8⁺; et
(c) ajouter des fragments d'antigènes associés à la sclérose en plaques (MS) et en option des cellules présentant des antigènes, où les antigènes associés à la MS sont sélectionnés dans le groupe consistant en protéine basique de la myéline (MBP), protéine protéolipidique (PLP) et glycoprotéine myéline oligodendrocyte (MOG) ; et
(d) répéter les étapes (c) une ou plusieurs fois.

2. Méthode selon la revendication 1, dans laquelle un ou plusieurs desdits fragments d'antigène associés à la MS comprennent une séquence exposée dans l'une quelconque des SEQ ID NOS : 1 - 4.

3. Méthode selon les revendications 1 ou 2, dans laquelle un ou plusieurs desdits fragments d'antigène associés à la MS comprennent des acides aminés 83-99 ou 151-170 de MBP.

4. Méthode selon l'une quelconque des revendications 1 à 3, dans laquelle l'étape (c) comprend en outre l'ajout de IL-2.

5. Méthode selon l'une quelconque des revendications 1 à 4, dans laquelle l'étape (c) comprend en outre l'ajout d'un mitogène.

6. Méthode selon la revendication 5, dans laquelle ledit mitogène est sélectionné dans le groupe consistant en phytohémagglutinine, conconavaline A, mitogène de phytolaque et anticorps monoclonaux a des CD3.


### SEQUENCE LISTING

<110> Baylor College of Medicine
<120> A Method for Increasing CD8+ Cytotoxic T Cell Responses and for Treating Multiple Sclerosis
<130> 05627.0010.00PC00
<140> Not available yet
   <141>
<150> US 60/512,212
   <151> 2003-10-17
<160> 5
<170> PatentIn version 3.2
<210> 1
   <211> 9
   <212> PRT
   <213> Synthetic
<400> 1
   Val Val His Phe Phe Lys Asn Ile Val
   1 5
<210> 2
   <211> 9
   <212> PRT
   <213> Synthetic
<400> 2
   Ser Leu Ser Arg Phe Ser Trp Gly Ala
   1 5
<210> 3
   <211> 9
   <212> PRT
   <213> Synthetic
<400> 3
   Asp Tyr Lys Ser Ala His Lys Gly Phe
   1 5
<210> 4
   <211> 9
   <212> PRT
   <213> Synthetic
<400> 4
   Lys Tyr Leu Ala Thr Ala Ser Thr Met
   1 5
<210> 5
   <211> 9
   <212> PRT
   <213> Synthetic
<400> 5
   Ser Thr Arg Gln Gly Pro Gln Glu Thr
   1 5